(19) Europäisches Patentamt / European Patent Office / Office européen des brevets

(11) **EP 3 501 388 A1**

(12) **EUROPEAN PATENT APPLICATION**

(43) Date of publication:
26.06.2019 Bulletin 2019/26

(51) Int Cl.:
*A61B 5/021* (2006.01)     *A61B 5/024* (2006.01)
*A61B 5/00* (2006.01)

(21) Application number: 17210496.0

(22) Date of filing: 22.12.2017

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA MD TN

(71) Applicant: **Biospectal SA**
**1006 Lausanne (CH)**

(72) Inventors:
• **SOLA I CAROS, Josep**
**2035 Corcelles (CH)**

• **PROENÇA, Martin**
**1723 Marly (CH)**
• **MUNTANÉ-CALVO, Enric**
**2000 Neuchâtel (CH)**
• **BONNIER, Guillaume**
**1023 Crissier (CH)**
• **SCHOETTKER, Patrick**
**1005 Lausanne (CH)**

(74) Representative: **reuteler & cie SA**
**Chemin de la Vuarpillière 29**
**1260 Nyon (CH)**

(54) **OPTICAL BLOOD PRESSURE MEASUREMENT METHOD AND SYSTEM**

(57)    Optical blood pressure measurement system (1) comprising an optical image acquisition system (4) and an acquisition system setup module (6), the image acquisition system comprising a camera (10) capable of capturing images of a pulsatile signal at a fingertip of a user. The image settings processing section comprises an image analysis section (14) configured to receive the sequence of images from the camera and to calculate metrics that are processed by the acquisition system setup section (16) to generate parameters for controlling the image acquisition system (4) configured to modify the quality of the pulsatile signal.

Fig. 1

EP 3 501 388 A1

## Description

### TECHNICAL FIELD

The present invention relates to a method and system for non-invasive measurement of blood pressure using optical signals.

### DESCRIPTION OF RELATED ART

One of the common non-invasive methods of measuring blood pressure on patients is based on inflating a cuff around the patient's upper arm or sometimes wrist, and using an oscillometric or ausculatatory technique to determine blood pressure values (systolic, diastolic, and/or pulse pressures). These methods are typically carried out by a health care practitioner in a clinic or with ambulatory medical equipment. Non-invasive blood pressure measurement devices such as automated oscillometric devices used by health practitioners are cumbersome and expensive, and access to professional equipment also means that blood pressure measurements may only be performed intermittently.

In order to perform blood pressure measurements more often and more economically, patients would benefit from a measurement system that does not require access to medical facilities or the presence of a health practitioner. In particular, there would be an advantage in providing a non-invasive system that enables personal monitoring and controlling of blood pressure in a simple manner without specialized equipment.

In WO/2016/138965 [4], a method for estimation of a blood pressure value based on the capture of a pulsatile signal (resulting from the pulsatile flow of blood) using a photoplethysmogram (PPG) sensor is disclosed. One of the devices proposed for capturing the pulsatile signal is a smartphone with a light source and light detector. The processing of a signal from a smartphone is however not specifically described in the foregoing publication.

Other blood pressure measurement systems using consumer electronic device sensors that have been proposed, as described in T. B. Plante, "Validation of the Instant Blood Pressure Smartphone App", JAMA Intern Med. 176:700-702, 2016 [2] are of insufficient accuracy.

For a non-invasive method based on an optical capture of the physiological pulsatile signal to generate reliable and accurate blood pressure measurement values, the quality and reliability of the captured signal is paramount.

There are however a number of difficulties in accurately optically capturing a pulsatile signal in a reliable and repeatable manner. Difficulties arise from various sources including the differences in morphology and physiology between patients, the varying positions of the patient's member relative to the camera or light source, the varying camera settings and properties, and the varying origin, intensity and color of light sources.

### SUMMARY OF THE INVENTION

In view of the foregoing, an object of the invention is to provide a non-invasive method and system for measurement of blood pressure values based on an optical capture of the physiological pulsatile signal, which is reliable, accurate and economical.

It is advantageous to provide a method and system for measurement of blood pressure values for personal use without requiring assistance of a medical practitioner.

It is advantageous to provide a method and system for measurement of blood pressure values using off-the-shelf consumer electronic devices, such as smartphones.

It is advantageous to provide a method and system for measurement of blood pressure values that is flexible and easily adaptable for different off-the-shelf consumer electronic devices, such as smartphones.

Objects of the invention are achieved by an optical blood pressure measurement system according to claim 1.

Objects of the invention are achieved by a method of measuring a blood pressure of a person using an optical blood pressure measurement system, according to claim 11.

Disclosed herein is an optical blood pressure measurement system comprising an optical image acquisition system and an acquisition system setup module, the image acquisition system comprising a camera capable of capturing images of a pulsatile signal at a fingertip of a user. The image settings processing section comprises an image analysis section configured to receive the sequence of images from the camera and to calculate metrics that are processed by the acquisition system setup section to generate parameters for controlling the image acquisition system, said parameters configured to enhance pulsatile signals from the sequence of images.

Also disclosed herein is a method of measuring a blood pressure of a person using an optical blood pressure measurement system comprising an optical image acquisition system and an acquisition system setup module, the image acquisition system comprising a camera capable of capturing images of a pulsatile signal at a fingertip of a user. The method is characterized by the steps of:

- receiving a sequence of images from the camera,
- analyzing parameters of the images affecting a quality of a pulsatile signal and calculating metrics that are used to generate acquisition system setup parameters,
- inputting said acquisition system setup parameters to the image acquisition system (4).

Pulsatile signals may be enhanced *inter alia* by any one or more of :

- maximizing a pulsation amplitude of one or multiple color (Red/Green/Blue (RGB)) channels within the region of interest ROI (AC amplitude),
- maximizing a homogenized color distribution,
- maximizing a temporal stability,
- minimizing a temporal-spatial dispersion,
- optimizing a trade-off between a measurement of the pulsation amplitude of one or multiple RGB channels within the ROI (AC amplitude) and a measurement of an average amplitude of one or multiple RGB channels within the ROI (DC amplitude).

The image acquisition system may further comprise a light source adjacent the camera.
In an advantageous embodiment, the metrics outputted by the image analysis section may comprise any one or more of:

- a color distribution, formed by a measurement of the distribution of colors within a region of interest,
- a spatial distribution formed by a measurement of the spatial distribution of one or more color channels within a region of interest,
- a DC value formed by a measurement of the average amplitude of one or multiple color channels within a region of interest,
- an AC value formed by a measurement of the pulsation amplitude of one or more color channels within a region of interest,
- a temporal-spatial distribution formed by a measurement of the spatial distribution of pulsation amplitudes of one or more color channels within a region of interest,
- a temporal-temporal stability formed by a measurement of the temporal stability of pulsation amplitudes of one or more color channels within a region of interest.

In an advantageous embodiment, the acquisition system setup section outputs image acquisition system parameters for control of the camera comprising any one or more of :

- working mode: the camera is set to acquire videos, or sequences of images;
- frame rate: a number of frames/images acquired per second;
- resolution: a number of pixels per frame;
- gamma correction: a luminance setting of the camera;
- auto focus: a setting on how the camera is allowed to perform autofocus operations;
- white balance: a setting on how the camera is allowed to perform white balance modifications;
- auto exposure: a setting on how the camera is allowed to perform auto exposure modifications;
- color correction gain: a fixed gain of color corrections;
- sensor sensitivity: a fixed sensitivity of the sensor;
- shutter opening time (or exposure time): a fixed shutter opening time.
- lens aperture: a possibly fixed lens aperture ("f/" value)

In an advantageous embodiment, the optical image acquisition system may comprise a light source and the acquisition system setup section is configured to generate control parameters for the light source.
In an embodiment, the acquisition system setup section outputs image acquisition system parameters for control of the light source comprising any one or more of:

- light intensity,
- light color,
- light intensity modulation,
- light color modulation,
- light on/off modulation.

The electronic device may comprise a microprocessor and a blood pressure measurement value program stored in a memory of the microprocessor and executable by a command initiated by a user via a use interface of the electronic device.

In an embodiment, the acquisition system setup section is configured to output image acquisition system parameters for control of the camera during an initialization or reset of the optical blood pressure measurement system.

In an embodiment, the acquisition system setup section is configured to output image acquisition system parameters for control of the camera at every measurement cycle.

In an embodiment, the acquisition system setup section is configured to output image acquisition system parameters for control of the camera continuously during a measurement process.

The blood pressure measurement system may comprise an electronic device incorporating the optical image acquisition system, and a blood pressure measurement program stored in a memory of the electronic device and executable in a microprocessor of the device.

In an advantageous embodiment, the electronic device is a smartphone.

In an embodiment, the blood pressure measurement program may be stored on a remote server and downloadable via a communications network in the electronic device.

In an advantageous embodiment, the parameters of the images affecting a quality of a pulsatile signal being analyzed may include any one or more of :

- a dispersion in the color domain of the red-green-blue (RGB) channels within a region of interest (ROI),
- a dispersion in the spatial domain of the red pixels within a ROI,
- a sum of all the red pixels within a ROI,
- a sum of the energy of cardiosynchronous pulses for all the RED pixels within a ROI.

Further objects and advantageous features of the invention will be apparent from the claims, from the detailed description, and annexed drawings, in which:

## BRIEF DESCRIPTION OF THE DRAWINGS

Figure 1 is a simplified block diagram illustrating a system for measurement of blood pressure values according to an embodiment of the invention;
Figure 2 is a schematic graph illustrating a pulsatile signal captured optically according to an embodiment of the invention.

## DETAILED DESCRIPTION OF EXEMPLARY EMBODIMENTS

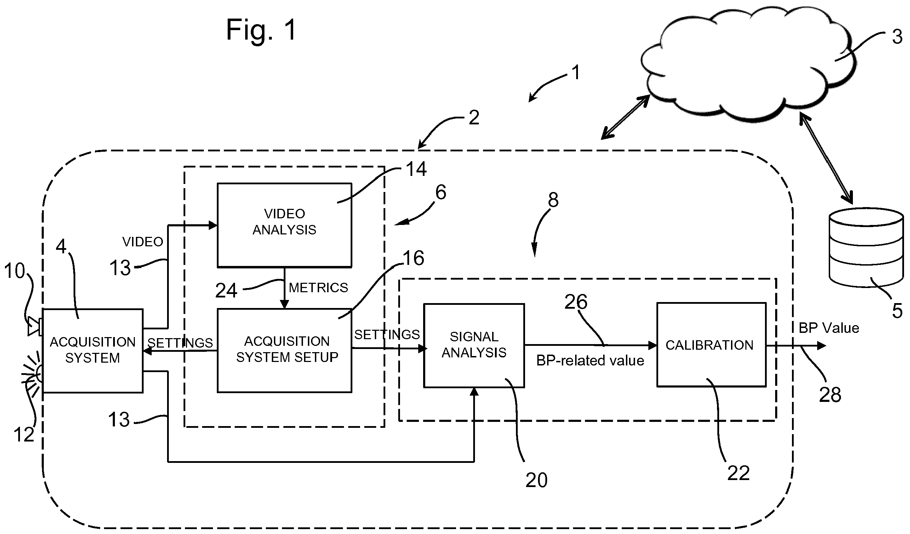

Referring to figure 1, a schematic simplified block diagram of an optical blood pressure measurement system 1 according to an embodiment of the invention is illustrated. The optical blood pressure measurement system comprises an electronic device 2, which in particular may be a commercial off-the-shelf smartphone having a blood pressure measurement program installed therein. The blood pressure measurement system 1 is configured to:

- acquire and process images of a person's finger or other member and extract therefrom a pulsatile signal, and
- analyze said pulsatile signal to generate a blood pressure measurement value.

The blood pressure measurement program is installed in a memory of the electronic device 2 and may be executed in a microprocessor of the electronic device when a blood pressure measurement process is instructed by a user via a user interface of the electronic device. The blood pressure measurement program may be pre-installed in the electronic device or downloaded from a remote server 5 via a global communications network 3. The blood pressure measurement program may also be updated whenever needed by downloading updates from the remote server 5 as is *per se* well-known in the art of computer programming.

The electronic device 2 and associated blood pressure measurement program comprises an optical image acquisition system 4, an acquisition system setup module 6 connected to the image acquisition system 4, and a measurement signal processing system 8 connected to the image acquisition system 4 and/or to the acquisition system setup module 6. The image acquisition system comprises comprising a camera 10 and optionally a light source 12 positioned adjacent and proximate a lens of the camera. The light source may serve as a flash for the camera, or as a torch that may be activated by the user as a general light source. In many conventional smartphones, the light source is typically placed in close proximity to the one or more lenses of the camera such that a person's finger may be placed simultaneously over both the camera lens 10 and the light source 12. The acquisition system setup module 6 comprises an image analysis section 14 and an acquisition system setup section 16.

The image acquisition system 4 may comprise a dedicated microprocessor for controlling operation of the camera 10 and optionally for operation of the light source 12 in conjunction with the camera 10, in particular to control various settings of the camera for the capture of continuous video images or sequences of images as *per se* well-known in the art of cameras.

A sequence of images such as a video captured by the camera 10 of the image acquisition system 4 is inputted into an image analysis section 14 of the acquisition system setup module 6. The image analysis section 14 analyses the sequence of images during execution of a blood pressure measurement process and outputs acquisition system metrics 24 to the acquisition system setup section 16. The acquisition system setup section 16 generates settings to control parameters of the image acquisition system 4.

Optical image acquisition system parameters that may be adjusted during the acquisition system setup include any one or more of the following:

*For the camera 10*

- Working mode: the camera is set to acquire videos, or sequences of images.
- Frame rate: number of frames/images acquired per second
- Resolution: number of pixels per frame
- Gamma correction: luminance setting of the camera
- Auto focus: setting on how the camera is allowed to perform autofocus operations
- White balance: setting on how the camera is allowed to perform white balance modifications
- Auto exposure: setting on how the camera is allowed to perform auto exposure modifications
- Exposure time: fixed exposure time of the camera
- Color correction gain: fixed gain of color corrections
- Sensor sensitivity: fixed sensitivity of the sensor
- Shutter opening time: fixed shutter opening time
- Lens aperture: fixed lens aperture "f/" value

*For the light source 12:*

- light intensity
- light color
- light intensity or color modulation
- light on/off modulation

*Example:* As an example, implementation on a smartphone using the operating system Android API 27, android.hardware.camera2 could include modification of the following parameters:

- COLOR_CORRECTION: setting on color correction algorithm
- CONTROL_AE_ANTIBANDING: setting on how the camera performs band pass filtering actions
- CONTRAST_CURVE: setting on camera contrast
- CONTROL_AE_MODE: setting on how the camera is allowed to perform auto exposure modifications, or to set manual exposure time values
- CONTROL_AF_MODE:CONTROL_AWB: setting on how the camera is allowed to perform white balance modifications
- CONTROL_CAPTURE: the camera is set to acquire videos, or sequences of images
- CONTROL_SCENNE: the camera is allowed to perform automatic scene optimization settings
- CONTROL_VIDEO_STABILIZATION: the camera is allowed to perform video stabilization actions
- EDGE_MODE: setting on how the camera performs edge enhancements actions
- INFO_SUPPORTED_HARDWARE_LEVEL: setting on the amount of control on camera settings
- LENS: setting on optical stabilization procedures
- LENS_APERTURE: setting on the desired lens aperture size
- NOISE_REDUCTION: settings on noise reduction algorithms

- SENSOR_EXPOSURE_TIME: setting on duration of exposure to light
- SENSOR_FRAME_DURATION: setting on frame rate
- TONEMAP_MODE_CONTRAST_CURVE: setting on contrast curve
- TONEMAP_MODE_GAMMA_VALUE: settings on luminance setting of the camera

The image analysis section 14 analyses the quality of the acquired sequence of images generated by the optical image acquisition system 4. Execution of the analysis may be performed continuously or at selected one or more of the following events:

- first use of the optical blood pressure measurement system 1;
- after a reset of the optical blood pressure measurement system 1;
- during an initiation phase of the optical blood pressure measurement system 1 for a specific electronic device 2;
- during execution of a blood pressure measurement process;
- subsequent to an operator prompted command;
- subsequent to a remote system prompted command.

The acquisition system setup module 6 may also output image acquisition control parameters to the image acquisition system 4 at the beginning of a blood pressure measurement process or throughout the duration of a blood pressure measurement process. The acquisition system setup module 6 may output image acquisition control parameters to the image acquisition system 4 at every blood pressure measurement process or intermittently at certain intervals of measurements.

The metrics generated by the image analysis section 14 are thus used to set commands for the image acquisition system 4 that seek to optimize the acquisition system setup for the properties of the electronic device, in particular of the optical image acquisition system 4. The image analysis section may further be configured to generate setting commands to optimize the acquisition system setup for a specific user, in particular to take into account the morphology and physiology of the patient (the patient's finger and arterial system). The image analysis section may further be configured to generate setting commands to optimize the acquisition system setup for specific use conditions environmental conditions, such as the intensity and color of environmental light, and the orientation and position of a patient's finger or other body member placed against the camera 10 of the image acquisition system 4.

The acquisition system setup section 16 may also be used to control the operation of the light source, in particular to control intensity, color or modulation of the light source to improve the robustness and reliability of the generated image. In particular, in a variant, the light source may be controlled to generate light that has a large red and/or green color component and a low blue color component in order to enhance the contrast of the tissue expanded or contracted by the changes of pressure in the arterial system.

The image analysis section 14 may also be configured to identify portions of the image that correspond to regions of interest. This may be based for instance on portions of the image that fall within a certain range of intensity, color or contrast, and to exclude portions of the image that have poor or low representations of pulsatile signals. By limiting measurements to regions of interest, a more robust and lower error reading of the pulsatile signal may be obtained.

As mentioned above, optimization of the acquisition system settings may be formed during an initialization phase, for instance when a user first places his/her fingertip in front of the camera during a lapse of time that may last a few seconds, or the optimization may be performed continuously during the video recording with a real time feedback loop. Optimization of the acquisition system settings may be performed once by the user when setting up his blood pressure measurement system for the first time, or when resetting the blood pressure measurement system, or at regular intervals, or at every measurement cycle or selected number of measurement cycles.

During the blood pressure measurement cycle, acquisition of the sequence of images will extend over a plurality of cardiac pulses, for instance 5-10 cardiac pulses depending on the time during which the user places his finger or other member on the camera.

The acquisition system setup section 16 may also be connected to the measurement signal processing system 8 to modify the sequence of images for analysis in the measurement signal processing system 8. The measurement signal processing system 8 use the acquisition system setup data to either to exclude information that is not within the regions of interest determined by the image analysis section 14 or to change certain values of the image such as white balance, contrast or color.

Regions of interest may be determined either as the entire image, which could form the region of interest, or pre-defined regions of interest can be used by dividing the image into two, three, four or more regions. Regions of interest may also be dynamic, for instance by changing the definition of regions of interest during the recording of an image according to the metrics calculated in each region of interest by the image analysis section 14. For each region of interest, a metrics vector may then be calculated, the metrics vector containing for instance a series of numbers (values) that represent the quality of the signals acquired in the region of interest. Examples of metrics for the regions of interest are presented below.

| Metric family | Metric description | Example |
|---|---|---|
| Color distribution | A measurement of the distribution of colors within the ROI | The dispersion (in the color domain) of the RGB channels within the ROI |
| Spatial distribution | A measurement of the spatial distribution of one or multiple RGB channels within the ROI | The dispersion (in the spatial domain) of the RED pixels within the ROI |
| DC values | A measurement of the average amplitude of one or multiple RGB channels within the ROI | Sum of all the RED pixels within the ROI |
| AC values | A measurement of the pulsation amplitude of one or multiple RGB channels within the ROI. | For all the RED pixels within the ROI, calculate the energy of cardiosynchronous pulses (pulses that are within a cardiac frequency band), and sum them. |
| Temporal-spatial dispersion | A measurement of the spatial distribution of pulsation amplitudes of one or multiple RGB channels within the ROI. | For all the RED pixels within the ROI, calculate the energy of cardiosynchronous pulses (pulses that are within a cardiac frequency band), and calculate its spatial dispersion. |
| Temporal-temporal stability | A measurement of the temporal stability of pulsation amplitudes of one or multiple RGB channels within the ROI. | For all the RED pixels within the ROI, calculate the energy of cardiosynchronous pulses (pulses that are within a cardiac frequency band), and calculate its stability in time |

The Acquisition System Setup section 16 receives as input the metrics vector calculated by the image analysis section 14, and determines the optimal settings of the image acquisition system 4. Each setting (see for instance system parameter setting examples provided above) can be calculated as a combination of metrics (see for instance metrics examples as provided above).

For instance, one can calculate the luminance (gamma correction) (for instance this parameter is called TONEMAP_MODE_GAMMA_VALUE in an Android operating system) to be set for the acquisition of the next frame (t+1) as:

$$Gamma\ Correction(t+1) =$$
$$Alpha * Gamma\ Correction(t) +$$
$$(1\text{-}Alpha) * GAMMA\_CORRECTION\_FACTOR *$$
$$(MAX\_AC\_VALUE - AC\ values) / MAX\_AC\_VALUE$$

Where:

- *Gamma Correction(t) was the luminance of the last acquired frame*
- *Alpha is a smoothing factor that ranges from 0 to 1 (Alpha = 1 does not allow any change of gamma correction, Alpha = 0 continuously changes gamma correction).*
- *GAMMA_CORRECTION_FACTOR is a weighting factor*
- *MAX_AC_VALUE is the maximum expected "AC Value"*
- *"AC Values" is the calculated AC Value during the last acquired frame (t).*

The above presents an example of closed-loop control of Gamma Correction, based on the calculated metrics "AC Values", however many other combinations of settings and metrics can be performed within the scope of the invention, for instance, one could also adapt parameters such as (examples from Android operating system):

COLOR_CORRECTION based on COLOR_DISTRIBUTION

COLOR_CORRECTION based on COLOR_DISTRIBUTION and

*TEMPORAL_SPATIAL_DISPERSION*

*CONTROL_AE_ANTIBANDING* based on *TEMPORAL_TEMPORAL_STABILITY*

*NOISE_REDUCTION* based on *TEMPORAL_TEMPORAL_STABILITY* and

*TEMPORAL_SPATIAL_STABILITY*

*TONEMAP_MODE_CONTRAST_CURVE* based on *AC_VALUES*

*SENSOR_EXPOSURE_TIME* based on *AC_VALUES* and *DC_VALUES*

And any additional and more sophisticated closed-loop correction algorithms could be implemented, rather than a simple smoothing-factor algorithm.

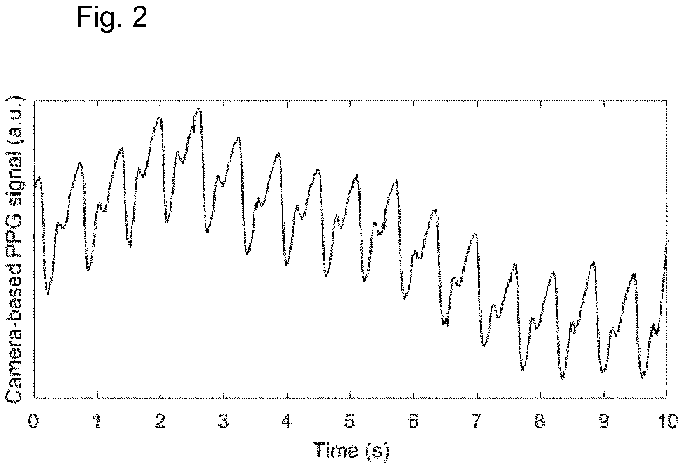

The measurement signal processing system 8 comprises a signal analysis section 20 that outputs a blood pressure related value 26, and a calibration section 22 that receives the blood pressure related value 26 and outputs a blood pressure measurement value 28. The calibration section 22 is optional and may for instance comprise a reference value that is received from an absolute blood pressure measurement device that allows to calibrate the values estimated by the optical blood pressure measurement system 1 with an external calibrated value. This may in particular be useful to allow the blood pressure measurement 1 to output estimations of absolute blood pressure measurement values as opposed to relative values. The signal analysis section receives the optimized sequences of images from the optical image acquisition system 4 and processes the pulsatile signals extracted from the sequence of images, as illustrated in figures 2a and 2b, and based thereon calculates a blood pressure measurement related value, by known methods for instance as described in publication WO 2016138965.

Advantages of the invention include:

- Easy use compare to a cuff-based approach
- No need for dedicated or specialized equipment - measurements can be performed by a consumer electronics device, in particular a smartphone device, compared to approaches that require the patient to buy dedicated hardware
- Measurements can be performed on any camera-based device since the device can automatically adapt to any smartphone or camera-system to provide optimized measurements

**List of features illustrated**

*Optical blood pressure measurement system 1*
*Computer program*
*Blood pressure measurement program*

*Electronic Device (Smartphone) 2*

***Optical image acquisition system 4***

*Camera 10*

*Light source 12*

*Output 13*

*Video*

***Acquisition system setup module 6***

***Image analysis section 14***

*Output 24*

*Metrics*

***Acquisition system setup section 16***


***Measurement signal processing system 8***

***Signal analysis section 20***

*Output 26*

*Blood pressure related value*

***Calibration section 22***

*Output 28*

*Blood pressure measurement value*


*Communications network 3*

*Remote computing system 5*

*Server*


PRIOR ART REFERENCES

[1] G. S. Stergiou, "Requirements for professional office blood pressure monitors", Journal of Hypertension, 30:537-542, 2012, doi: 10.1097/HJH.0b013e32834fcfa5

[2] T. B. Plante, "Validation of the Instant Blood Pressure Smartphone App", JAMA Intern Med. 176:700-702, 2016, doi: 10.1001/jamainternmed.2016.0157

[3] J. Solà, "Continuous non-invasive monitoring of blood pressure in the operating room: a cuffless optical technology at the fingertip", Proceedings BMT2016, Basel, Switzerland, 2016, doi: 10.1515/cdbme-2016-0060

[4] M. Proença, "Method, apparatus and computer program for determining a blood pressure value" WO Patent PCT/EP 2015/063765. 2015

[5] L. A. Critchley, "Assessment of Trending Ability of Cardiac Output Monitors by Polar Plot Methodology", Journal of Cardiothoracic and Vascular Anesthesia 25:536-546, 2011, doi: 10.1053/j.jvca.2011.01.003.


**Claims**

1. **Optical blood pressure measurement system** (1) comprising an optical image acquisition system (4) and an acquisition system setup module (6), the image acquisition system comprising a camera (10) capable of capturing images of a pulsatile signal at a fingertip of a user, **characterized in that** the image settings processing section comprises an image analysis section (14) configured to receive the sequence of images from the camera and to calculate metrics that are processed by the acquisition system setup section (16) to generate parameters for controlling the image acquisition system (4), said parameters configured to enhance pulsatile signals from the sequence of images.

2. Blood pressure measurement system according to any preceding claim, wherein the metrics outputted by the image analysis section (14) comprise any one or more of:

- a color distribution, formed by a measurement of the distribution of colors within a region of interest,
- a spatial distribution formed by a measurement of the spatial distribution of one or more color channels within a region of interest,
- a DC value formed by a measurement of the amplitude of one or multiple color channels within a region of interest,
- an AC value formed by a measurement of the pulsation amplitude of one or more color channels within a region of interest,
a temporal-spatial distribution formed by a measurement of the spatial distribution of pulsation amplitudes of one or more color channels within a region of interest,
- a temporal-temporal stability formed by a measurement of the temporal stability of pulsation amplitudes of one or more color channels within a region of interest.

3. Blood pressure measurement system according to any preceding claim, wherein the acquisition system setup section (16) outputs image acquisition system parameters for control of the camera (10) comprising any one or more of :

- working mode: the camera is set to acquire videos, or sequences of images;
- frame rate: a number of frames/images acquired per second;
- resolution: a number of pixels per frame;
- gamma correction: a luminance setting of the camera;
- auto focus: a setting on how the camera is allowed to perform autofocus operations;
- white balance: a setting on how the camera is allowed to perform white balance modifications;
- auto exposure: a setting on how the camera is allowed to perform auto exposure modifications;
- color correction gain: a fixed gain of color corrections;
- sensor sensitivity: a fixed sensitivity of the sensor;
- shutter opening time (or exposure time): a fixed shutter opening time.
- lens aperture: a fixed lens aperture ("f/" value)

4. Blood pressure measurement system according to any preceding claim, wherein the optical image acquisition system (4) comprises a light source (12) and the acquisition system setup section (16) outputs image acquisition system parameters for control of the light source (12) comprising any one or more of :

- light intensity,
- light color,
- light intensity modulation,
- light color modulation,
- light on/off modulation.

5. Blood pressure measurement system according to any preceding claim, wherein the acquisition system setup section (16) is configured to output image acquisition system parameters for control of the camera (10) during any one or more of the following events:

- an initialization or reset of the optical blood pressure measurement system (1);
- at every measurement cycle.

6. Blood pressure measurement system according to any preceding claim, wherein the acquisition system setup section (16) is configured to output image acquisition system parameters for control of the camera (10) continuously during a measurement process.

7. Blood pressure measurement system according to any preceding claim comprising an electronic device (2) incorporating the optical image acquisition system (4), and a blood pressure measurement program stored in a memory of the electronic device and executable in a microprocessor of the device.

8. Blood pressure measurement system according to the preceding claim, wherein the electronic device (2) is a smartphone.

9. Blood pressure measurement system according to the preceding claim, wherein the blood pressure measurement program is stored on a remote server and downloadable via a communications network in the electronic device.

10. Blood pressure measurement system according to any preceding claim, wherein the image acquisition system further comprises a light source (12) adjacent the camera (10).

11. **Method of measuring a blood pressure** of a person using an optical blood pressure measurement system (1) comprising an optical image acquisition system (4) and an acquisition system setup module (6), the image acquisition system comprising a camera (10) capable of capturing images of a pulsatile signal at a fingertip of a user, **characterized by:**

 - receiving a sequence of images from the camera,
 - analyzing parameters of the images affecting a quality of a pulsatile signal and calculating metrics that are used to generate acquisition system setup parameters,
 - inputting said acquisition system setup parameters to the image acquisition system (4).

12. The method of the preceding claim, wherein the parameters of the images affecting a quality of a pulsatile signal being analyzed include any one or more of :

 - a dispersion in the color domain of the red-green-blue (RGB) channels within a region of interest (ROI),
 - a dispersion in the spatial domain of the red pixels within a ROI,
 - a sum of all the red pixels within a ROI,
 - a sum of the energy of cardiosynchronous pulses for all the RED pixels within a ROI.

13. The method of any preceding claim, wherein the calculated metrics include any one or more of :

 - a color distribution, formed by a measurement of the distribution of colors within a region of interest,
 - a spatial distribution formed by a measurement of the spatial distribution of one or more color channels within a region of interest,
 - a DC value formed by a measurement of the amplitude of one or multiple color channels within a region of interest,
 - an AC value formed by a measurement of the pulsation amplitude of one or more color channels within a region of interest,
 - a temporal-spatial distribution formed by a measurement of the spatial distribution of pulsation amplitudes of one or more color channels within a region of interest,
 - a temporal-temporal stability formed by a measurement of the temporal stability of pulsation amplitudes of one or more color channels within a region of interest.

14. The method of any preceding claim, wherein an acquisition system setup section (16) of the blood pressure measurement system (1) outputs image acquisition system parameters for control of the camera (10) comprising any one or more of :

 - working mode: the camera is set to acquire videos, or sequences of images;
 - frame rate: a number of frames/images acquired per second;
 - resolution: a number of pixels per frame;
 - gamma correction: a luminance setting of the camera;
 - auto focus: a setting on how the camera is allowed to perform autofocus operations;
 - white balance: a setting on how the camera is allowed to perform white balance modifications;
 - auto exposure: a setting on how the camera is allowed to perform auto exposure modifications;
 - color correction gain: a fixed gain of color corrections;
 - sensor sensitivity: a fixed sensitivity of the sensor;
 - shutter opening time or exposure time: a fixed shutter opening time.

- lens aperture: a fixed lens aperture "f/" value

15. The method of any preceding claim, wherein an acquisition system setup section (16) of the blood pressure measurement system (1) outputs image acquisition system parameters for control of a light source of the blood pressure measurement system (1) comprising any one or more of :

- light intensity,
- light color,
- light intensity modulation,
- light color modulation,
- light on/off modulation.

Fig. 1

Fig. 2

Europäisches
Patentamt

European
Patent Office

Office européen
des brevets

# EUROPEAN SEARCH REPORT

Application Number

EP 17 21 0496

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2015/150096 A1 (KONINKL PHILIPS NV [NL]) 8 October 2015 (2015-10-08) | 1,3-7, 10,11, 14,15 | INV. A61B5/021 A61B5/024 A61B5/00 |
| Y | * page 5, line 10 - page 6, line 18 * <br> * page 7, line 5 - page 8, line 26 * <br> * page 10, line 12 - page 12, line 10 * <br> * figures 1-2 * <br> ----- | 8,9 | |
| Y | US 2016/360980 A1 (SINHA TUHIN [US] ET AL) 15 December 2016 (2016-12-15) <br> * paragraphs [0017], [0024] - [0028] * <br> * paragraphs [0030] - [0032], [0035] - [0038] * <br> * paragraphs [0050], [0062], [0065] * <br> * figures * <br> ----- | 8,9 | |
| X | US 2014/155759 A1 (KAESTLE SIEGFRIED WALTER [DE] ET AL) 5 June 2014 (2014-06-05) <br> * paragraphs [0041] - [0044] * <br> * paragraphs [0051] - [0061], [0082] * <br> * figures * <br> ----- | 1,2,4, 10-13 | |
| | | | **TECHNICAL FIELDS SEARCHED (IPC)** |
| A | US 2014/276089 A1 (KIRENKO IHOR OLEHOVYCH [NL] ET AL) 18 September 2014 (2014-09-18) <br> * paragraphs [0074] - [0079] * <br> * figure 4 * <br> ----- | 1-15 | A61B |
| A | US 2017/105638 A1 (KULACH CHRISTOPHER J [CA]) 20 April 2017 (2017-04-20) <br> * paragraphs [0018] - [0024] * <br> * paragraphs [0028], [0035] - [0036] * <br> ----- | 1-15 | |

The present search report has been drawn up for all claims

| Place of search | Date of completion of the search | Examiner |
|---|---|---|
| The Hague | 7 June 2018 | Bataille, Frédéric |

EPO FORM 1503 03.82 (P04C01)

## ANNEX TO THE EUROPEAN SEARCH REPORT
## ON EUROPEAN PATENT APPLICATION NO.

EP 17 21 0496

This annex lists the patent family members relating to the patent documents cited in the above-mentioned European search report.
The members are as contained in the European Patent Office EDP file on
The European Patent Office is in no way liable for these particulars which are merely given for the purpose of information.

07-06-2018

| Patent document cited in search report | Publication date | Patent family member(s) | Publication date |
|---|---|---|---|
| WO 2015150096  A1 | 08-10-2015 | CN    106163390  A<br>EP      3125755  A1<br>JP  2017512578  A<br>US  2017014087  A1<br>WO  2015150096  A1 | 23-11-2016<br>08-02-2017<br>25-05-2017<br>19-01-2017<br>08-10-2015 |
| US 2016360980  A1 | 15-12-2016 | CN    107847156  A<br>US  2016360980  A1<br>WO  2016205414  A1 | 27-03-2018<br>15-12-2016<br>22-12-2016 |
| US 2014155759  A1 | 05-06-2014 | BR 112015012718  A2<br>CA      2893324  A1<br>CN    104768452  A<br>EP      2928360  A1<br>JP      6054543  B2<br>JP  2016503327  A<br>RU  2015126594  A<br>US  2014155759  A1<br>WO  2014087310  A1 | 11-07-2017<br>12-06-2014<br>08-07-2015<br>14-10-2015<br>27-12-2016<br>04-02-2016<br>13-01-2017<br>05-06-2014<br>12-06-2014 |
| US 2014276089  A1 | 18-09-2014 | BR 112015022112  A2<br>CN    105050492  A<br>EP      2967376  A1<br>JP  2016514986  A<br>RU  2015143949  A<br>US  2014276089  A1<br>WO  2014140148  A1 | 18-07-2017<br>11-11-2015<br>20-01-2016<br>26-05-2016<br>20-04-2017<br>18-09-2014<br>18-09-2014 |
| US 2017105638  A1 | 20-04-2017 | US  2017105638  A1<br>US  2017105682  A1 | 20-04-2017<br>20-04-2017 |

EPO FORM P0459

For more details about this annex : see Official Journal of the European Patent Office, No. 12/82

## REFERENCES CITED IN THE DESCRIPTION

**Patent documents cited in the description**

- WO 2016138965 A

- EP 2015063765 W


**Non-patent literature cited in the description**

- **T. B. PLANTE.** Validation of the Instant Blood Pressure Smartphone App. *JAMA Intern Med.,* 2016, vol. 176, 700-702
- **G. S. STERGIOU.** Requirements for professional office blood pressure monitors. *Journal of Hypertension,* 2012, vol. 30, 537-542

- **J. SOLÀ.** Continuous non-invasive monitoring of blood pressure in the operating room: a cuffless optical technology at the fingertip. *Proceedings BMT2016,* 2016
- **L. A. CRITCHLEY.** Assessment of Trending Ability of Cardiac Output Monitors by Polar Plot Methodology. *Journal of Cardiothoracic and Vascular Anesthesia,* 2011, vol. 25, 536-546